# EUROPEAN PATENT APPLICATION

(11) **EP 2 455 084 A2**
(43) Date of publication of application: **23.05.2012**
(21) Application number: 10800012.6
(22) Date of filing: 13.07.2010
(51) Int. Cl.: A61K 31/704, A61P 9/12, A23L 1/30, A61K 9/14

(54) **ANTIHYPERTENSIVE COMPOSITION CONTAINING A GINSENOSIDE-ENRICHED FRACTION**

(30) Priority: 16.07.2009 KR 20090065018
(71) Applicant: CJ CheilJedang Corporation, Jung-gu Seoul 100-749 (KR)
(72) Inventor: KIM, Hye-Jin, Suwon-si Gyeonggi-do 442-082 (KR); KIM, Se-Gon, Seoul 156-791 (KR); LEE, Jin-Hee, Seoul 132-020 (KR); PARK, Seok-Jun, Seoul 135-904 (KR); KIM, Yeong-Eun, Seoul 152-050 (KR); KWON, Oran, Seoul 138-767 (KR)
(74) Representative: Ford, Hazel
(86) International application number: PCT/KR2010/004536
(87) International publication number: WO 2011/008001

(57) **Abstract**

The present invention related to an antihypertensive composition containing a fraction enriched with ginsenosides of ginseng. The composition according to the present invention increases the production of nitric oxide through the activation of nitric oxide synthase, and thus exhibits superior antihypertensive effects through the vasodilatory activity. Consequently, the composition of the present invention can be used as a composition for preparing functional health foods for preventing and treating hypertension and various cardiovascular diseases caused by complications of hypertension.

## Description

### [Technical field]

The present invention relates to an antihypertensive composition containing a ginsenoside-enriched fraction. More specifically, the present invention relates to the antihypertensive composition containing ginsenoside, where its effective dose is between 0.1-95 weight%.

### [Background Art]

According to the Korean National Health % Nutrition Examination Survey 2005, approximately 27% of Korean 20 years and older men, 21% of 20 years and older women and more than half of adults over 60 years of age have high blood pressure. Hypertension shows no observed symptoms, but shows high recognition rate such as arthritis and dental caries. The recognition rate of hypertension in adults over 30 years old is increasing, which was 27% in 1998, 36% in 2001 and 60% in 2005.

Hypertension is affected by various causes including hereditary factors and environmental factors, and occurs by (1) increase of blood volume due to excessive accumulation of salt in the body; (2) decrease of artery elasticity and increase of peripheral blood vessel- resistance. Not only hypertension itself is a problem, but also it brings more burden to the heart as it needs to contract more than necessary. Also, as the blood vessels are distributed all over the body, hypertension can cause diseases in various organs all over the body. For example, in Korea, the contribution rates of hypertension causing cardiac disorder and cerebrovascular disorder are 21 % and 35% respectively. Hypertensive patients should take medicines and improve lifestyle to manage their health, as the blood pressure tends to increase over time. However, considering financial investigations the patients should take on medication and side effects, the prevention of prehypertension is essential.

Ginseng is a food including ginsenoside, alkaloid, polyacetylene, polysaccharide, oligosaccharide, oligopeptide, flavonoid, lipid, vitamin and minerals. Ginsenoside is the main functional component of the ginseng, and is classified as terpinoid and saponin. Approximately 30 types of Ginsenoside structures have been reported. Most research have used 98% pure mono ginsenoside in their experiments, and therefore it is not reasonable to use the data to support the functionality of ginseng, ginseng extract or ginseng fraction. Although there have been reports using extracts, the number of reports is little. Also, the standardisation information of ginsenosides has not been provided, and therefore there are limited database on the functionality of ginseng, ginseng extract and ginseng fraction.

The inventors of the present invention aimed to solve the mentioned problems occurring in the production of antihypertensives. Therefore, the inventors repeated carefully designed experiments to produce an antihypertensive composition containing a ginsenoside-enriched fraction by decreasing the blood pressure through relaxation of the blood vessels due to activation of nitric oxide synthase and the increased production of nitric oxide.

### [Disclosure]

### [Technical problem]

Therefore the aim of the present invention is to provide the ginseng ginsenoside-enriched fraction. Also, another aim is to provide an antihypertensive composition containing ginsenoside-enriched fraction.

### [Technical solution]

The aim of the present invention is achieved by providing an antihypertensive composition containing ginsenoside-enriched fraction including ginsenoside Rg1 and Rb1.

The mentioned ginsenoside-enriched fraction contains 10-15 weight %, preferably 12 weight % of ginsenoside Rg1; and also contains 5-10 weight %, preferably 6 weight % of ginsenoside Rb1.

The antihypertensive composition from this invention is characterised by preferably containing 0.1 to 95 weight % of ginsenoside-enriched fraction.

Also, the mentioned ginsenoside-enriched fraction is characterised by the following manufacturing steps; solvent extraction by adding alcohol to dried root hair of ginseng; heating concentration of the mentioned alcohol extraction of ginseng; diluting the mentioned concentration adding distilled water and passing through absorbent resin; passing through 20 v/v% alcohol after passing through distilled water, and eluting to 30-40 v/v% alcohol.

In other words, the ginseng alcohol extraction is manufactured by adding C1-C4 lower alcohol to the dried root hair of ginseng. Preferably, the ginseng alcohol extraction is manufactured by adding 70% alcohol 5-20 times heavier than the sample, preferably 15 times, preferably extracting at 70°C for 12-48 hours, preferably for 24 hours. The ginseng alcohol extraction is heat concentrated to 50-100brix, preferably 80brix. The concentration is then diluted with 2-10 times heavier amount of distilled water, preferably, 5 times. Then the diluted concentration is passed through Diaion HP-20 resin for saponin absorption, followed by passing through with distilled water which the amount is 2-10 times, preferably 5 times amount of the mentioned resin, in order to remove the unabsorbed components. Then, 20v/v% of alcohol which the amount is 2-10 times, preferably 5 times larger than the resin volume is passed through to remove non-saponin components, followed by eluting to 30-40 v/v% alcohol concentration, in order to obtain ginsenoside-enriched fraction.

Vascular endothelium is known to liberate various components that play a role in contraction and relaxation of the blood vessel therefore regulating the tension of vascular smooth muscle. Among these components, the representative molecule is nitric oxide (NO) which is an endothelium-derived relaxing factor. NO is produced from vascular endothelium, smooth muscle cells, circulating lymphocytes, nerve cells and the central nervous system and other areas of the body. NO can be used as a marker for understanding the pathophysiology of hypertension, and it has been reported that alterations of NO dependent-vasodilation was linked with the induction of hypertension. NO is produced from the amino acid L-arginine, catalysed by nitric oxide synthase (NOS).

Therefore in the present invention, the vasodilative effect of a composition containing ginsenoside -enriched fraction is investigated by measuring the production of nitric oxide within the vascular endothelial cells and the activity of nitric oxide synthase. As a result, it was found that the composition containing ginsenoside-enriched fraction induced the production of NO from vascular endothelial cells causing vasodilation, and the increase of the protein expression of endothelial NO synthase (eNOS) when added to human vascular endothelial cell line HUVEC. Spontaneously hypertensive rats (SHR) was used as an animal model of hypertension. This animal model shows hypertension from week 5-6, and its systolic blood pressure is 280-200mmHg. It was found from the experiment administering the antihypertensive composition containing ginsenoside-enriched fraction into SHR animals, that the blood pressure decreases due to vasodilation caused by increased nitric oxide production from activation of nitric oxide synthase.

According to the results above, the present antihypertensive composition containing ginsenoside-enriched fraction is characterised by being a pharmaceutical composition or food composition which can be used for the prevention or treatment of hypertension. The present antihypertensive composition can be manufactured into various forms including powder, granules, tablet, capsule, syrup and drink form containing ginsenoside-enriched fraction as an active component. As far as it does not affect the active component, various carriers used for health function foods, for example diluting agents, additives, stabilizers, safeners, emulsifiers, can be used in the process of manufacturing.

### [Advantageous effects]

As described above, the present antihypertensive composition containing ginsenoside-enriched fraction is effective in decreasing the blood pressure by increasing the production of nitric oxide due to increase of activation and expression of nitric oxide synthase in the blood vessel. Also, this composition is effective in preventing hypertension and effectively provides hypotensive health function foods.

### [Description of drawings]

Figure 1 is a graph showing the amount of ginsenoside Rg1 and Rb1 from ginseng alcohol extraction.
Figure 2 is a graph showing the amount of ginsenoside Rg1 and Rb1 from ginsenoside-enriched fraction according to the present invention.
Figure 3 is a graph showing the amount of nitric oxide (NO) produced from the human vascular endothelial cell when the antihypertensive composition containing ginsenoside-enriched fraction is added.
Figure 4 shows images and a graph illustrating the expression of endothelial nitric oxide synthase (eNOS) from the human vascular endothelial cell when the antihypertensive composition containing ginsenoside-enriched fraction is added.
Figure 5 shows a graph illustrating the hypotensive effects of the antihypertensive composition containing ginsenoside-enriched fraction administered into spontaneously hypertensive rats (SHR).
Figure 6 shows a graph illustrating the activation of nitric oxide synthase in spontaneously hypertensive rats (SHR) administered with the antihypertensive composition containing ginsenoside-enriched fraction.

### [Mode for invention]

### Example 1: Manufacturing of the ginsenoside-enriched fraction from the present invention

### 1-1: Manufacturing of ginseng alcohol extraction

The ginseng alcohol extraction is manufactured by adding 70% alcohol which is 15 times heavier than the sample to the dried root hair of ginseng, followed by 24 hours of extraction at 70°C.

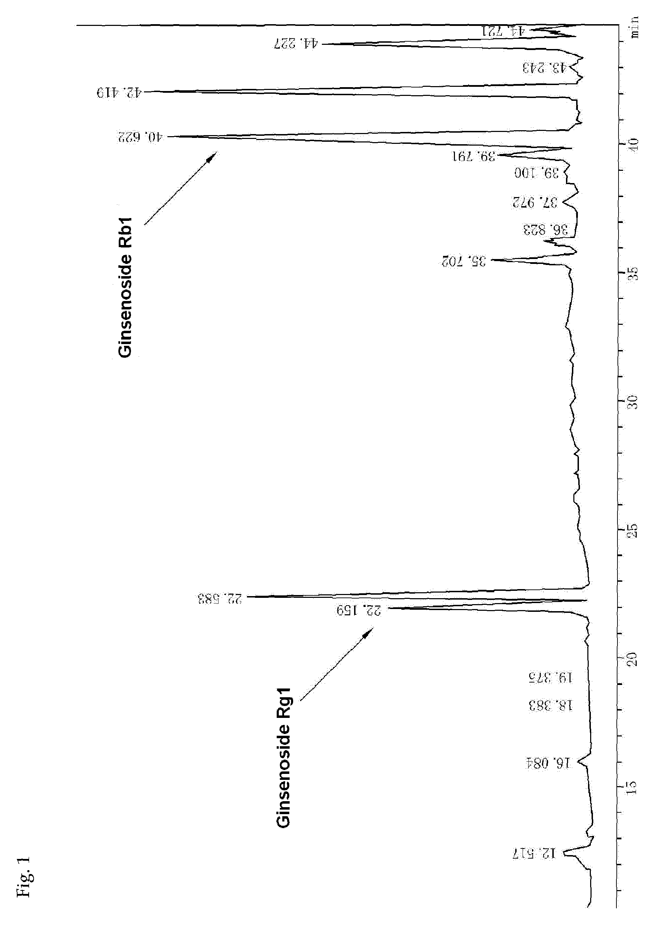

The amount of Rg1 and Rb1 was measured by high performance liquid chromatography (HPLC) from the obtained ginseng alcohol extraction, and the results are shown in Table 1 and Figure 1.

### 1-2: Manufacturing the ginsenoside-enriched fraction

The mentioned ginseng alcohol extraction from Example 1-1 was heat concentrated to 80 brix. Distilled water which was five times heavier than the concentration was added for dilution, and then the diluted concentration was passed through Diaion HP-20 resin for saponin absorption followed by passing through distilled water which was 5 times the volume of the mentioned resin.

Then, 20 v/v% of alcohol which the volume was 5 times larger than the resin volume was passed through to remove non-saponin components followed by elution into 30-40 v/v% alcohol to obtain ginsenoside-enriched fraction.

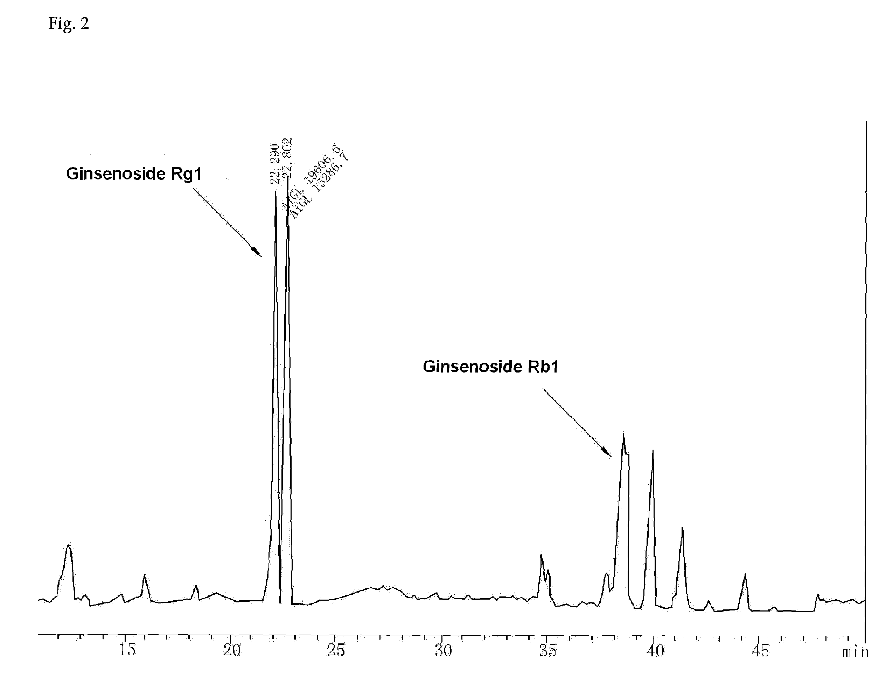

The amount of ginsenoside Rg1 and ginsenoside Rb1 was obtained from the mentioned fraction through high performance liquid chromatography (HPLC) and the results are shown in Table 1 and Figure 2.

**Table 1.**

| | Amount of ginsenoside Rg1 | Amount of ginsenoside Rb1 |
|---|---|---|
| Ginseng alcohol extraction | 0.7 weight % | 2.0 weight % |
| Ginsenoside-enriched fraction | 12.0 weight% | 6.0 weight % |

The present antihypertensive composition containing ginsenoside -enriched fraction can be manufactured into the forms described below. However, the purpose of these examples is only to illustrate the present invention, not limiting the scope of the invention thereto in any way.

### Example 2: Manufacturing the antihypertensive composition in a tablet form containing ginsenoside-enriched fraction according to the present invention.

The obtained ginsenoside-enriched fraction 25mg from Example 1 was mixed with 270mg microcrystalline cellulose and 200mg dextrin. Further, 2.5mg magnesium and 2.5mg hydroxypropylmethylcellulose was added to the mixture for the manufacturing.

### Example 3: Manufacturing the antihypertensive composition in a capsule form containing ginsenoside-enriched fraction according to the present invention.

500mg of ginsenoside-enriched fraction obtained from Example 1 was filled into a soft gelatine capsule for the manufacture.

### Example 4: Manufacturing the antihypertensive composition in a drink form containing ginsenoside-enriched fraction according to the present invention.

5g of ginsenoside-enriched fraction obtained from Example 1 was dissolved in 50mL of water. Then as a supplement, 15mL of vitamin and oligosaccharide and 1.5mL of preserved agent was added and topped up with water to make 100mL of the drink.

### Experiment 1: Measurement of nitric oxide (NO) from human vascular endothelial cells treated with the antihypertensive composition containing ginsenoside-enriched fraction according to the present invention.

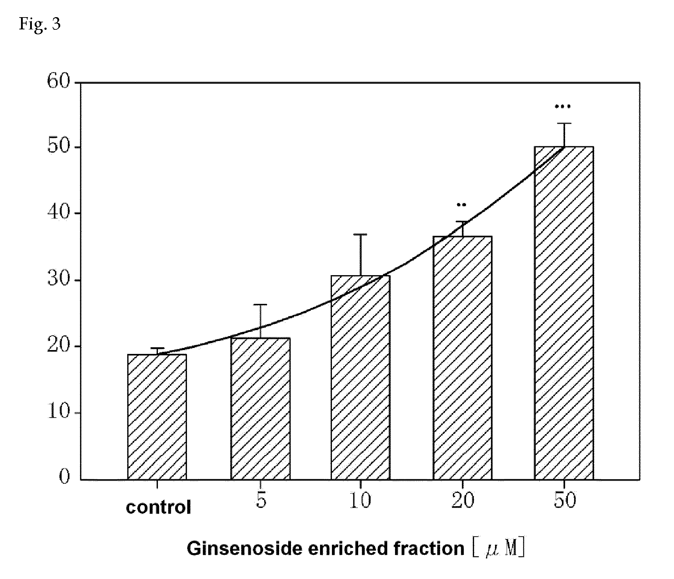

The amount of nitric oxide (NO) production in the human vascular endothelial cell treated with ginsenoside-enriched fraction manufactured using the method described in Example 1 is illustrated in Figure 3.

Experiment was performed by culturing human vascular endothelial cells (HUVEC) in 24 well plate and then manufacturing various concentrations of the antihypertensive composition containing ginsenoside-enriched fraction. Then, 0.5mL of ginsenoside-enriched fraction of which the final concentration was 0-50µm was added to the wells and the plate was incubated for 30 minutes under 5% CO₂ at 37°C. After the incubation, the medium was moved to a 0.4mL tube for centrifugation, and the supernatant was collected to measure the nitric oxide production using Griess reagent kit. The amount of nitric oxide was quantified by converting to protein amount in µg.

As a result illustrated in Figure 3, the amount of nitric oxide increased dose-dependently. The amount of measured protein was 18.8nmol/µg when 0 µM of ginsenoside-enriched fraction was added, but the amount of protein increased to 50.34nmol/µg when 50 µM of the fraction was treated. The amount of nitric oxide production increased dose-dependently as the ginsenoside-enriched fraction was treated at 5,10,25,50 µM concentration. The amount of nitric oxide significantly increased at 25 µM and 50 µM compared with 0 µM (p<0.01 and p<0.001 respectively). Further repeated experiments showed the same results.

### Experiment 2: Measurement of endothelial nitric oxide synthase (eNOS) expression from human vascular endothelial cells treated with the antihypertensive composition containing ginsenoside-enriched fraction according to the present invention.

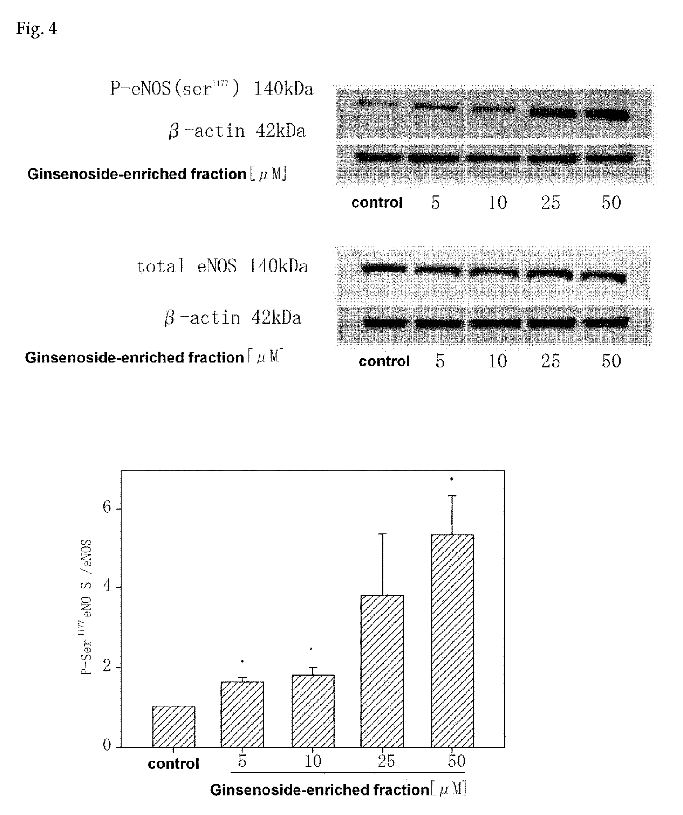

The expression of endothelial nitric oxide synthase (eNOS) production in the human vascular endothelial cell treated with ginsenoside-enriched fraction manufactured using the method described in Example 1 was measured and illustrated in Figure 4.

The measurement of nitric oxide synthase from the vascular endothelial cells was performed by using Western blotting. Same amount of vascular endothelial cells were cultured on culture plates and 0, 0.09, 0.26, 0.52, 0.77J/cm² of laser was irradiated and cultured for 24 hours. The adhered cells were dissolved using protease, and the total protein was extracted with buffers. The cells were scrapped, and centrifuged at 14,000g, 4°C for 15 minutes. SDS-PAGE membrane was incubated with monoclonal anti- human eNOS antibody and polyclonal anti- mouse actin antibody protein. The test was repeated for three times, and densitometer analysis was used for quantification and the results were expressed as percentage over actin.

As a result shown in Figure 4, the protein expression of nitric oxide synthase increased as the concentration of ginsenoside-enriched fraction increased. Especially, when the concentration of ginsenoside-enriched fraction was 5,10,25,50µM, the expression of nitric oxide synthase was significantly increased compared to the control (p<0.01).

### Example 3: Measuring the hypotensive effects of the antihypertensive composition containing ginsenoside-enriched fraction administered in spontaneously hypertensive white rats (SHR).

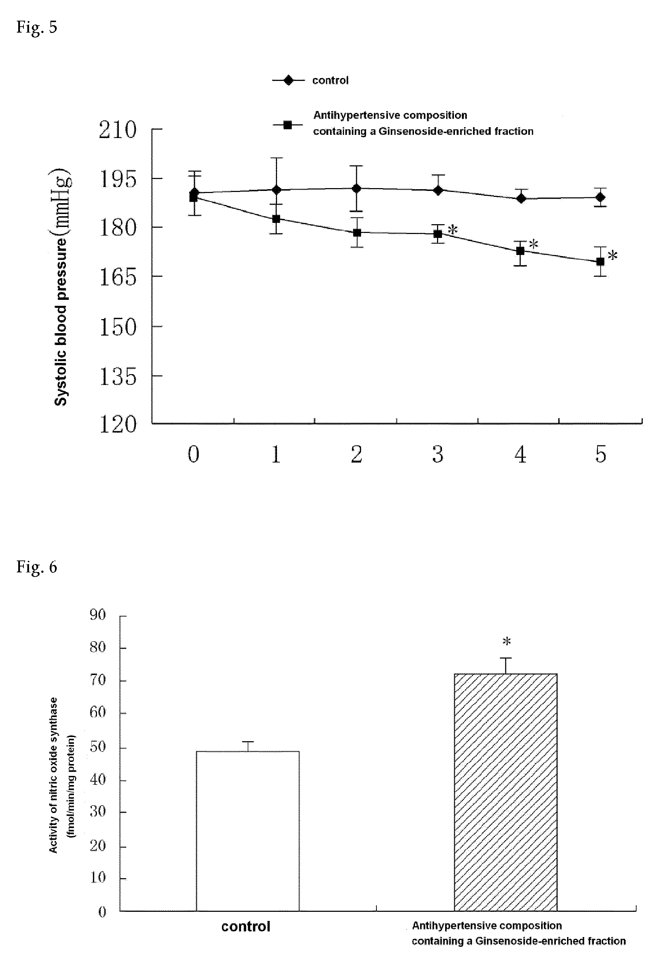

Measurement of hypotensive effects has been shown in Figure 5, from the spontaneous hypertensive rats treated with the antihypotensive composition containing ginsenoside-enriched fraction manufactured using the method described in example 1.

The 16 weeks old spontaneously hypertensive rats (SHR) were purchased and were housed at a normal laboratory conditions at 22±1°C, relative humidity 65±5%, 12hr light/dark cycle, and food and water were fed *ad libitum.* After a week's adaptation period, the rats were grouped in two groups. The control group were fed with AIN-93 basic diet, and the experimental group were fed with antihypertensive composition containing ginsenoside-enriched fraction in addition to the basic diet for 5 weeks. The systolic blood pressure and diastolic blood pressure was measured from the animals' tail using the tail-cuff method. In order to measure a stable blood pressure, the animals were adapted to the environment from 2weeks before the actual experiment, by fixing them in a frame in a constant-temperature water tank for 10 to 15 minutes, and the measurement was done at a designated time (14:00-16:00).

As a result shown in Figure 5, the systolic blood pressure of the control group and the group administered with the composition containing hypertensive ginsenoside-enriched fraction changed to 200mmHg and 170mmHg respectively after 5 weeks of administration, where the systolic blood pressure was 189-190mmHg in both groups before the experiment. The difference of systolic blood pressure was significantly different from week 3 (p<0.05), and the difference became larger towards the end of the experiment.

### Experiment 4: Measuring the activity of nitric oxide synthase after administration of the antihypertensive composition containing ginsenoside-enriched fraction administered in spontaneously hypertensive white rats (SHR).

The experimental results on measurement of nitric oxide synthase activity in spontaneously hypertensive white rats treated with antihypertensive composition containing ginsenoside-enriched fraction is illustrated in Figure 6. The main artery was removed and its tissue was homogenised in Tris buffer (50mM Tris, 0.1 % mercaptoethanol, 0.1mM EDTA, 0.1mM EGTA, 2mM leupeptin, 1mM PMSF, 1mM pepstatin, pH7.4) using the tissue homogeniser. The activity of nitric oxide synthase is measured through the amount of conversion of [³H]-L-arginine to [³H]-L-citrulline. The tissue homogenate (60µg) was added to the reaction solution (1mM MgC1₂, 1mM CaCl₂, 1mM DTT, 2mM NADPH, 3µM TBH₄, 3 µM FAD, 3 µM FMN, 10µg/mL Calmodulin, 2µCi/ml [³H]-L-arginine) for reaction at 37°C for 20 minutes. The reaction was stopped using 500µL of stop solution (20mM HEPES, 2mM EGTA, 2mM EDTAm pH 5.5). The reaction solution was passed through Dowex 50W-X8 (Na⁺ form) column and [³H]-L-citrulline was extracted for quantification using the liquid scintillation counter. Non-specific reaction was determined by [³H]-L-citrulline extracted from the reaction solution containing 0.1mM L-NAME. As a result, the activity of nitric oxide synthase measured from the main artery in animals treated with an antihypertensive composition containing ginsenoside-enriched fraction (72fmo1/min/mg) was significantly increased compared with control group (49fmol/min/mg) (p<0.05).

### [Industrial applicability]

The present invention is valuable for health supplement functional food industry as it provides antihypertensive composition containing ginsenoside-enriched fraction which is effective in lowering the blood pressure.

## Claims

1. Antihypertensive composition containing ginsenoside-enriched fraction including ginsenoside Rg1 and Rb1 as an active component.

2. Antihypertensive composition of claim 1, **characterised by** ginsenoside Rg1 being 10-15 weight percent (%) of ginsenoside-enriched fraction.

3. Antihypertensive composition of claim 1, **characterised by** ginsenoside Rb1 being 5-10 weight percent (%) of ginsenoside-enriched fraction.

4. Antihypertensive composition of claim 1, where the effective dose of ginsenoside-enriched fraction is 0.1-95 weight percentage (%).

5. Antihypertensive composition of claim 1, **characterised by** the following procedures to manufacture ginsenoside-enriched fraction; adding alcohol to the dried root hair of ginseng for solvent extraction; performing heating concentration of ginseng alcohol extraction; diluting the concentration by adding distilled water and passing the diluent through absorption resin; passing dilstilled water and 20v/v% of alcohol through, and eluting into 30-40v/v% of alcohol.

6. Antihypertensive composition of claim 1, **characterised by** being a pharmaceutical composition or food composition.

7. Antihypertensive composition of claim 1, **characterised by** being one of the forms such as powder, granule, tablet, capsule, syrup, and drink.
